# EUROPÄISCHE PATENTSCHRIFT

(11) **EP 1 534 659 B1**
(45) Veröffentlichungstag und Bekanntmachung des Hinweises auf die Patenterteilung: **12.03.2008**
(21) Anmeldenummer: 03792015.4
(22) Anmeldetag: 25.08.2003
(51) Int. Cl.: C07C 67/03, C11C 3/00

(54) **UM- UND VERESTERUNG VON FETTS UREN UND TRIGLYCERIDEN DURCH DISPERGIEREN UND DISPERSIONSVERFAHREN ZUR HERSTELLUNG VON FETTSÄUREMETHYLESTERN**
TRANSESTERIFICATION AND ESTERIFICATION OF FATTY ACIDS AND TRIGLYCERIDES BY DISPERSION AND DISPERSION METHOD FOR THE PRODUCTION OF FATTY ACID METHYLESTERS
TRANSESTERIFICATION ET ESTERIFICATION D'ACIDES GRAS ET DE TRIGLYCERIDES PAR DISPERSION ET PROCEDE DE DISPERSION POUR LA PRODUCTION DE METHYLESTERS D'ACIDES GRAS

(30) Priorität: 23.08.2002 AT 12622002
(43) Veröffentlichungstag der Anmeldung: 01.06.2005
(73) Patentinhaber: Gapes, Richard, 1133 Wien (AT); Baumgartner, Hans, 1220 Wien (AT)
(72) Erfinder: Gapes, Richard, 1133 Wien (AT); Baumgartner, Hans, 1220 Wien (AT)
(74) Vertreter: Sonn & Partner Patentanwälte
(86) Internationale Anmeldenummer: PCT/AT2003/000242
(87) Internationale Veröffentlichungsnummer: WO 2004/018405

(56) Entgegenhaltungen:
- WO-A-99/26913
- "Römpps Chemie-Lexikon" 1981 , NEUMÜLLER, OTTO-ALBRECHT , STUTTGART - GERMANY XP002266102 Bd.2, 8.Aufl. Seite 986 -Seite 987 Seite 1127 -Seite 1130

## Beschreibung

Die Erfindung betrifft ein Verfahren zur basisch oder sauer katalysierten Ver- und Umesterung von Ölen und Fetten, nämlich den Estern des Glyzerins mit Fettsäuren sowie den Fettsäuren selbst, durch Einbringen von Methylalkohol oder anderen niedrigen Alkoholen in das flüssige Ausgangsprodukt.

Für viele Anwendungen ist es erwünscht. Fette durch Umestern verschiedenen technischen Notwendigkeiten anzupassen, insbesondere wenn es um die Herstellung von Motortreibstoffen auf biologischer Basis geht.

Aus verschiedenen pflanzlichen Ölen und Fetten, sind derartige Verfahren zur Umesterung bekannt und führen über eine mehrstufige Verfahrenskette, die schrittweise diskontinuierlich vollzogen wird, schließlich im wesentlichen zu Glyzerin, Wasser und dem gewünschten Methylester der Fettsäuren, die ursprünglich mit dem Glyzerin verestert waren. Es ist auch möglich, statt des Methylalkohols einen anderen niedrigen Alkohol, beispielsweise Ethanol, Propanol, Butanol und in Sonderfällen selbst Pentanol zu verwenden und so zu den entsprechenden Estern zu kommen, doch wird aus Kostengründen und wegen der einfacheren Reaktion zumeist Methylalkohol verwendet. Mit zunehmender Kettenlänge wird die saure Katalyse wirksamer und damit zu einer ernsthaften Alternative.

Im Ausgangsmaterial enthaltene Verunreinigungen sollten soweit entfernt werden, dass sie keine verfahrenstechnische Probleme verursachen. Eine vorherige Reinigung des Öls vermindert die Nachteile, die durch die Verunreinigungen entstehen: Nebenreaktionen, höherer Chemikalienverbrauch, langsamere Umsetzung, etc..

Probleme macht hingegen der langsame Verfahrensablauf, der eine Behandlung des ursprünglichen Öls mit Methylalkohol, zumeist basisch katalysiert in einem großvolumigen Rührkessel über Zeiten abläuft, die in Stunden zu messen sind. Gefolgt wird dieser Reaktionsschritt von einem Abtrennschritt, der wiederum in einem großen Absetzbehälter stattfindet, wobei gegebenenfalls Glyzerin im Bodenbereich und das zu etwa oder über 90 % umgesetzte Halbprodukt darauf aufschwimmend anfällt, allerdings eventuell auch erst nach einigen Stunden des Absetzens.

Das aufschwimmende Halbfertigprodukt wird erneut in einen Rührkessel übergeführt und erneut mit Methylalkohol und Lauge umgesetzt, wobei nach einigen Stunden eine etwa 99 %-ige oder höhere Umsetzung, bezogen auf das Ausgangsmaterial, erreicht wird.

Auch dieses Produkt muss noch dekantiert werden, auch dies erfolgt in großen Absetzbehältern, in denen das Produkt aufschwimmt und endlich abgezogen wird. Erst dieses Produkt wird anschließend der Endreinigung unterzogen.

Die Veresterung von Fettsäuren funktioniert analog zu der oben beschrieben Umesterung, jedoch findet die Reaktion langsamer statt, und es wird Wasser anstatt Glyzerin als Nebenprodukt erzeugt. Die Reaktion wird normalerweise mit Säure katalysiert. Die Erfindung bezieht sich genauso auf die Ver- und Umesterung von Fettsäuren und Ölen bzw. Fetten als Gemisch miteinander sowie mit andern Komponenten.

Zusammenfassend ist festzuhalten, dass die meisten heutzutage kommerziell verwendeten Verfahren auf oben beschriebene Weise funktionieren. Prinzipiell basieren alle diesen Verfahren auf den Frühentwicklungen, die zwecks Herstellung von Fettsäuremethylestern als Rohstoffe für chemische Industrie ausgearbeitet wurden (US 2360844, US 2383632).

Die Erfindung besteht nun darin, dass in dem (den) als flüssiges Ausgangsprodukt vorliegenden, gegebenenfalls mit freien Fettsäuren verunreinigten Öl(en) bzw. Fett(en) ein technisch reiner bzw. technisch reine niedrige(r) Alkohol(e) in Gegenwart eines basischen oder sauren Katalysators dispergiert wird.

Beim erfindungsgemäßen Verfahren wird die Dispersion mit normalen Dispergiergeräten hergestellt. Diese Geräte sind dazu gebaut, vorübergehend stabile Dispersionen herzustellen. Man war allgemein der Annahme, dass sich die im Dispergiergerät gebildete Mischung nur sehr langsam trennt, und damit die Absetzzeit der Glycerinphase enorm lang wird. Es konnte nun nachgewiesen werden, dass bei der Wahl des richtigen Dispergierwerkzeugs eine sehr schnelle Phasentrennung erfolgt.

Die Erfindung wird im folgenden, unter ausführlicher Behandlung des Standes der Technik, und Bezugnahme auf die Zeichnung, näher erläutert, wobei
die Fig. 1 bis 5 verschiedene Tröpfchengrößenverteilungen und
die Fig. 6 eine erfindungsgemäß verwendbare Dispergiermaschine zeigen.

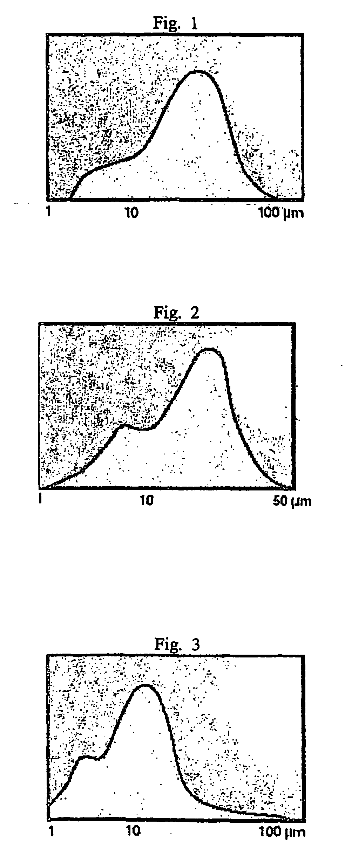

Bedeutsam für die Durchführung der Erfindung ist die Wahl der Tröpfchengröße. Fig. 1 bis 5 (Quelle: Firmenangaben) zeigen die Verteilung der Tröpfchengröße bei Verwendung von unterschiedlichen Werkzeugen des gleichen Maschinentyps. Ein wichtiges Charakteristikum eines Dispergiergerätes ist, dass die Tröpfchengröße bzw. dessen Spektrum unabhängig vom Durchsatz bis zu ihrer Höchstleistung konstant bleibt. Diese Tatsache ist insbesondere bedeutsam, wenn weiter unten auf das Patent WO 99/26913 A1 eingegangen wird.

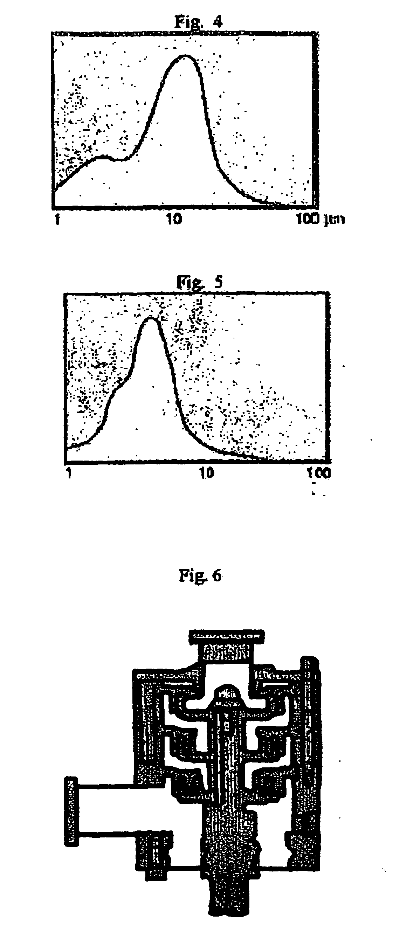

In einer Versuchsreihe können auf einfache Weise geeignete Werkzeuge für die Ver- und Umesterung ermittelt werden. Die Dispersion soll bevorzugt eine Tröpfchengröße (Durchmesser) von über 3, bevorzugt über 5 µm, und von unter 50, bevorzugt unter 15 µm, aufweisen. Verteilungen gemäß den Fig. 3 und 4 können besonders vorteilhaft verwendet werden.

Fig. 6 zeigt den grundsätzlichen Aufbau eines Dispergiergerätes. Vergleicht man diesem Aufbau mit den Geräten die in WO 99/26913 A1, DE 199 08 978 A1, EP 0 249 463 A2, US 4 668 439 A, DE 196 38 460 A1 und DE 100 43 644 A1 beschrieben sind, kann man leicht den Unterschied erkennen. In den oben genannten Druckschriften werden entweder Statikmischer (oder eine Ableitung davon) oder ganz normale Rührkessel verwendet.

Ein weiteres besonderes Merkmal der Erfindung, das sie von den oben genannten Patenten unterscheidet, ist die Tatsache, dass in mehreren davon von einer Mehrstufigkeit des Verfahrens ausgegangen wird, während beim erfindungsgemäßen Dispersionsverfahren das Produkt in hinreichender Qualität (nach der einschlägigen EN) bereits nach der ersten Stufe vorliegt.

Ein weiterer wesentlicher Unterschied zu WO 99/26913 A1 ist, dass Dispergiergeräte (wenn nicht aus anderen verfahrenstechnischen Gründen gefordert) bei normalem atmosphärischen Druck arbeiten. Ein Überdruck wie in WO 99/26913 A1 beschrieben, ist nicht notwendig.

Nachdem die generellen Unterschiede zwischen dem erfindungsgemäßen Verfahren und den Dokumenten WO 99/26913 A1, DE 199 08 978 A1, DE 199 08 978 A1, US 4 668 439 A, DE 196 38 460 A1, DE 100 43 644 A1 aufgezeigt wurden, soll nun im Einzelnen auf die Unterschiede beziehungsweise die Verbesserung, dass das angemeldete Verfahren bringt eingegangen werden. Vorab soll allerdings noch auf den Unterschied zwischen Dispersion und Emulsion eingegangen werden. Beim Dispergieren geht es um die Vergrößerung der Phasengrenzfläche zwischen zwei ineinander unlöslichen Flüssigkeiten. Wird dieser Vorgang durch den Zusatz von oberflächenaktiven Substanzen bewirkt, spricht man von Emulgieren, für das in der Regel gänzlich andere Gesetzmäßigkeiten gelten (siehe: Marko Zlokarnik, Rührtechnik, Theorie und Praxis, Springer Verlag 1999). Der Vollständigkeit halber sollen nun definitionsgemäß die beiden Verfahren beschrieben werden:

### Dispergieren:

Dispergieren ist ein physikalischer Vorgang, bei dem eine Mischung aus zwei oder mehreren Stoffen entsteht, die sich dadurch auszeichnet, dass die Stoffe nicht ineinander löslich sind und die Verteilung des dispergierten Stoffes im Dispersionsmittel sehr fein ist, so dass die Dispersion wie eine homogene, stabile Mischung erscheint. Dispersion ist der Oberbegriff für alle Mischungen, die sich aus einem Trägermedium und dem darin dispergierten Stoff zusammensetzen.

### Emulgieren:

Unter Emulgieren wird ein besonderer Dispersionsvorgang verstanden, wobei die erzeugt Dispersion auf Grunde einer niedrigen Oberflächenspannung stabil ist und die verteilten Tröpfchen sich nicht abtrennen lassen. Häufig werden auch oberflächenaktive Substanzen zur Steigerung der Stabilität der entstandenen Emulsion eingesetzt. Die oberflächenaktiven Substanzen wirken dabei als Emulgatoren.

Die Unterschiede der Erfindung zu den einzelnen vorveröffentlichten Druckschriften:

### Zur WO 99/26913 A1:

Die WO 99/26913 A1 Weg beschreibt im wesentlichen ein Verfahren das mit statischen Mixern arbeitet. (Seite 8 Zeile 16, 17) der Statikmixer besteht aus einem Rohr das mit Kugeln oder diversen anderen Materialien gefüllt ist. (Seite 8 Zeile 23 bis 25). Auf Seite 9 sind ersatzweise Geräte oder Stoffe angeführt, die offensichtlich den Statikmixer ersetzen oder unterstützen sollen. Als Emulgator ist üblicherweise ein Stoff und kein Gerät zu verstehen. "Emulgatoren sind Hilfsstoffe, mit deren Hilfe zwei miteinander nicht mischbare Flüssigkeiten (z.B. Wasser in Öl) zu einer stabilen, homogenen Masse, genannt Emulsion, verarbeitet werden können. Es gibt künstliche und natürlich vorkommende Emulgatoren. Sie besitzen in ihrem Molekül einen wasserliebenden (hydrophilen) und einen fettliebenden (lipophilen) Bereich. Diese Molekülshuktur befähigt sie dazu, eine Wechselwirklung zwischen Wasser und Öl einzugehen und eine mikroskopisch kleine Feinstverteilung des Wassers zu ermöglichen. Für die Emulsion Margarine nutzt man als Emulgatoren überwiegend Mono- und Diglyceride auf Basis von pflanzlichen Ölen und Fetten sowie pflanzliches Lecithin." (Deutsches Margarineinstitut). Der auf Seite 9 Zeile 12 erwähnte Turbulator ist ein Gerät, das aus der Wärmetechnik kommt und zu Deutsch "Turbulenzerzeuger" bedeutet. Es ist ähnlich aufgebaut wie ein Statikmixer, jedoch werden keine Stoffe vermischt sondern der Übergang von Wärme zum Rohr wird begünstigt (siehe: A. Klaczak Heat and Mass Transfer Springer Verlag Heidelberg 1996).

Auf Seite 14 Zeile 10 bis 13 wird wiederholt das die Reaktionsstrecke aus einem mit Kugeln gefüllten Rohr besteht In Zeile 22 und 23 wird auf den hohen Druck hingewiesen, der bei diesem Verfahren notwendig ist. Auf Seite 16 Zeile 9 bis 12 wird der so genannte "dynamische Emulgator" ebenfalls als Statikmixer beschrieben, der in diesem Fall aus einem gebogenen Rohr besteht. Wiederholt wird auch auf Seite 16 Zeile 30 darauf hingewiesen dass es sich bei dem genannten Verfahren um ein Hochdruckverfahren handelt.

Aus den Patentansprüchen drei, vier, fünf, sieben und acht ist erkennbar, dass hier eine Abart eines Statikmixers beschrieben wird die mit der gegenständlichen Erfindung nichts zu tun hat. Dies ist auch aus den Zeichnungen Figur 1 und Figur 2 leicht erkennbar.

Der Vollständigkeit halber sei erwähnt, dass sich unter besonders günstigen Umständen auch mit einem Statikmixer Dispersionen herstellen lassen. Das Problem dabei ist allerdings, dass es notwendig ist, dass die Dehnung lange genug anhält. Das Problem besteht somit darin, dass eine Durchsatzsteigerung die Schergeschwindigkeiten erhöht und gleichzeitig die Verweilzeit im Mischer herabsetzt. Die erreichbare Tröpfchengröße ist jedoch in einem Statikmixer u.a. auch eine Funktion der Verweilzeit. Dieser Nachteil eines statischen Mischers tritt bei einem Dispergiergerät nicht auf (siehe: Marko Zlokarnik, Rührtechnik, Theorie und Praxis, Springer Verlag 1999).

Auf die anderen Punkte die in WO 99/26913 A1 wie Reinigung, Waschung, Destillation usw. angeführt sind wird hier nicht eingegangen, da es sich um Verfahren handelt, die allgemein bekannt sind und allgemein angewandt werden.

### Zur DE 199 08 978 A1:

In der DE 199 08 978 A1 wird ein Verfahren beschrieben, dass die Ver- und Umesterung in einem Arbeitsschritt durchführt. Üblicherweise wird für die Veresterung als Katalysator Schwefelsäure, für die Umesterung Kaliauge oder Natronlauge verwendet, weil die verschiedenen Katalysatoren sich beim Mischen gegenseitig neutralisieren und das Verfahren aus mindestens zwei Verfahrensschritten besteht. Das Dokument beschreibt die simultane Ver- und Umesterung in einem Reaktionsschritt unter Verwendung von Sulfonsäure als einzigem Katalysator.

Von der apparativen Ausgestaltung her ist das Verfahren als Kaskade konventioneller "Mixer - Settler" zu sehen. Somit stellt DE 199 08 978 A1 kein naheliegendes Vorbild Für die gegenständliche Erfindung dar.

### Zur EP 0 249 463 A2:

Zu dieser Druckschrift ist zu vermerken das sie mit der erfindungsgemäßen Dispersionsmethode im Kern nichts zu tun hat Sie beschreibt eine Methode der Vorveresterung und Umesterung in zwei getrennten Schritten. In Figur 2 ist ein ganz normales, wie vielfach angewandtes Rührwerk abgebildet. Diese Art der Rührkessel werden zur Zeit üblicherweise für die Ver- und Umesterung gebraucht und gehören zum Stand der Technik. Nach Figur 1 ist zu erkennen, dass es sich zuerst um eine Veresterung und danach um eine Umesterung handelt. Hierbei unterscheidet sich die EP 0 249 463 A2 grundlegend von der DE 199 08 978 A1, die mit den gleichen Katalysator die Ver- und Umesterung in einem Schritt durchführt. Es ist wichtig darauf hinzuweisen, dass die Zeitangaben, die in Figur 3 bis 6 angegeben sind, durch das erfindungsgemäße Verfahren zumindest halbiert werden können. (Dies bezieht sich auch auf die Veresterung, die wesentlich langsamer als die Umesterung abläuft). Die EP 0 249 463 A2 beschreibt somit ein Verfahren, wie es dem Stand der Technik entspricht ohne Neuigkeiten aufzuweisen.

### Zur US 4 668 439 A:

Die US 4 668 439 A unterscheidet sich vom erfindungsgemäßen Verfahren in mehreren wesentlichen Punkten. Zum ersten handelt es sich dabei um ein Verfahren das mit gasförmigen Methanol arbeitet. Das Verfahren findet unter hohen Temperaturen statt (230 bis 240°C) (Tabelle 1, Seite 9 und 10). Wie im Beispiel 1 (Seite 7 und 8) ausgeführt wird findet die Umesterung in einem ganz normalen Rührbehälter statt. Im Unterschied zum Dispersionsverfahren wird eine Reaktionszeit von 3,75 Stunden angegeben (Seite 7 Zeile 57). Wie in Seite 11, Punkt 7 erwähnt wird findet das Verfahren unter Überdruck statt. Damit unterscheidet es sich ebenfalls vom Dispersionsverfahren.

### Zur DE 196 38 460 A1:

Die DE 196 38 460 A1 bezieht sich im wesentlichen nicht auf die Ver- und Umesterung sondern auf eine möglichst schnelle und effiziente Trennung der Glycerinphase vom erzeugten Ester. Wie auf Seite 4 Zeile 45 bis 63 beschrieben wird in dieser Erfindung der Umstand genutzt, dass der Ester in nah superkritischen Medien wesentlich leichter löslich ist als Trigyceride oder partiale Glyceride. Die Herstellung des Esters mittels eines Dispergiergerätes ist weder in den Patentansprüchen von S. 7 und 8 zu lesen, noch in der Zeichnung Fig. 1 erkennbar. Die in Beispiel 3 und Beispiel 4 angegebene Methode mit einem festen Katalysator die Umesterung durchzuführen geht sogar der Dispersionsmethode diametral entgegen. Mit der Dispersionsmethode ist es nicht möglich, einen festen Katalysator zu verwenden.

Die in Beispiel 1 und 2 verwendete Methode ist eine gängige Form der Umesterung, es wird jedoch hier hauptsächlich auf die Abtrennung des Esters Bezug genommen. Auch hier könnte die Dispersionsmethode bei der Herstellung des Esters hilfreich sein. Extraktionsmethoden mit superkritischen Medien sind durchaus üblich (Koffeinfreier Kaffee und andere) werden jedoch derzeit in der Herstellung von Fettsäuremethylester noch nicht angewandt.

### Zur DE 100 43 644 A1:

Die DE 100 43 644 A1 beschreibt ein Verfahren zur kontinuierlichen Herstellung von Biodiesel mit sogenannten "Mikroreaktoren". Diese Mikroreaktoren, wie sie in Fig. 1 eingezeichnet sind, haben mit der erfindungsgemäßen Dispersionsmethode nichts gemeinsam. Die Unterschiede sind dadurch begründet das es sich offensichtlich um ein Verfahren handelt, dass ähnlich wie WO 99/26913 A1 mit statischem Mischer arbeitet. Die Ähnlichkeit der Verfahren zwischen DE 100 43 644 A1 und WO 99/26913 A1 ist wesentlich größer als zwischen DE 100 43 644 A1 und der Erfindung. Weiters handelt es sich um einen mehrstufigen Prozess (Seite 4 Zeile 50) während es sich bei der Erfindung um einen einstufigen Prozess handelt.

Wie diese Stellungnahme zeigt, haben die angeführten Druckschriften mit der gegenständlichen Erfindung nichts zu tun. Es wird beansprucht, dass die Ver- und Umesterung mittels eines Dispergierverfahrens durchgeführt wird. Vorteile des erfindungsgemäßen Verfahrens sind, dass die Ver- und Umesterung mittels geeigneter Werkzeuge in jeweils einer Stufe durchgeführt werden kann; dass durch die Auswahl der geeigneter Werkzeuge die Erzeugung einer stabilen Dispersion vermieden wird; und dass durch die Auswahl der geeigneter Werkzeuge die Phasentrennung bereits in weniger als 30 Minuten zu 90 Prozent abgeschlossen ist. Es wird durch dieses Verfahren, in Kombination mit passenden Trenngeräten (Koaleszenztrenner, Seperatoren, Schrägklärer, usw.) möglich, dass die Trennung in situ erfolgen kann.

Auf Grund eigener Recherchen und auf Grund von Aussagen durch den weltweit führenden Hersteller für Dispersionsgeräte wurde das Verfahren bis jetzt noch nirgendwo angewandt. Der Grund dafür ist, das bei falscher Werkzeugwahl die gewünschte Trennung nicht mehr erfolgt. Das Verfahren ist somit neu.

Bei der Überlegung, die Reaktion der Ver- bzw. Umesterung zu beschleunigen, muss die Tatsache berücksichtigt werden, dass es sich bei der Reaktion um eine heterogene chemische Reaktion handelt. Der Grund dafür ist der Umstand, dass die beiden Reaktanten (Fett bzw. Öl bzw. Fettsäure und Methanol) miteinander äußerst begrenzt mischbar sind. Prinzipiell stellen die heterogenen Reaktionen eine Kombination zwischen chemischen Reaktionen und Massentransportphänomenen dar, sie sind allerdings aus folgenden Gründen noch komplizierter:

Da sich die Reaktionskomponenten in zwei Phasen befinden, muss der Massentransfer zur Grenzfläche als Transport in zwei Gegenrichtungen betrachtet werden.

Hydrodynamische Phänomene spielen bei diesen Systemen eine wesentlich wichtigere Rolle.

In meisten Fällen finden chemische und physikalische Prozeßschritte gleichzeitig statt und können nicht getrennt analysiert werden.

Die Löslichkeit der Komponenten in beiden Phasen muss betrachtet werden, da dadurch bestimmt ist, ob die Reaktion in nur einer oder in beiden Phasen stattfindet.

Man kann offensichtlich eine heterogene chemische Reaktion durch Verbesserung des Phasenkontaktes erheblich beschleunigen. Dies hat sich schon bei unserem ersten Versuch bewahrheitet, wobei wir die zur Herstellung von Fettmethylester üblichen Reaktanten zusammenfügten und statt mit einem Propeller- oder anderem im chemischen Betrieb üblichen Rührer zu rühren, die Komponenten mit einer Dispergiermaschine intensiv zu bearbeiten. Die durch die intensiv Bearbeitung hervorgerufene Vergrößerung der Phasenkontaktoberfläche trug dazu bei, dass die Reaktion der Ver- bzw. Umesterung in sehr kurzer Zeit praktisch vollständig zu Ende ging.

Es besteht somit das erfindungsgemäße Verfahren darin, die in flüssiger Form vorliegen Fette bzw. Öle, die gegebenenfalls mit Fettsäuren gemischt sind, in Gegenwart eines basischen oder sauren Katalysators mit technisch reinem Methylalkohol so zu versetzten, dass der Phasenkontakt zwischen den beiden nicht mischbaren Reaktanten (Fett bzw. Öl bzw. Fettsäuren und Methanol) auf geeignete Weise so vergrößert wird, dass die Reaktion der Ver- bzw. Umesterung in kürzester Zeit vollständig ablaufen kann. Die verwendete Apparatur ist in der Lage eine Dispersion mit einer Tröpfchengröße von ca. 1 µm herzustellen.

Die durch die Herstellung einer Dispersion zu erwartende lange Absetzzeit der einzelnen Phasen findet überraschenderweise nicht statt. Bei der Umesterung ist bereits nach einer Zeit von 10 Minuten die Glycerinphase deutlich zu erkennen, und die Trennung ist bereits zu 90 % vollzogen.

Die Dispersionsvorrichtung muss in der Lage sein, durch Aufbringen von Scherkräften die gewünschten Tröpfchengröße bzw. Tröpfchengrößenverteilungen zu erreichen.

## Patentansprüche

1. Verfahren zur basisch oder sauer katalysierten Ver- und/oder Umesterung von Fettsäuren und/oder Ölen und/oder Fetten, nämlich den Estern des Glyzerins mit Fettsäuren, durch Einbringen von niedrigen Alkoholen, insbesondere Methylalkohol, in das flüssige Ausgangsprodukt, **dadurch gekennzeichnet, dass** in dem flüssigen Ausgangsprodukt mindestens ein technisch reiner niedriger Alkohol in Gegenwart eines basischen oder sauren Katalysators mittels eines Dispergiergeräts unter Vermeidung der Erzeugung einer stabilen Dispersion dispergiert wird, **dadurch gekennzeichnet, dass** die Phasentrennung in weniger als 30 Minuten zu 90 % abgeschlossen ist.

2. Verfahren nach Anspruch 1, **dadurch gekennzeichnet, dass** die Phasentrennung in weniger als 10 Minuten zu 90 % abgeschlossen ist.

3. Verfahren nach einem der Ansprüche 1 bis 2, **dadurch gekennzeichnet, dass** die Dispersion eine Tröpfchengröße von ca. 1 µm aufweist.

4. Verfahren nach einem der Ansprüche 1 bis 2, **dadurch gekennzeichnet, dass** die Dispersion eine Tröpfchengröße von über 3 µm, bevorzugt über 5 µm, aufweist.

5. Verfahren nach Anspruch 4, **dadurch gekennzeichnet, dass** die Dispersion eine Tröpfchengröße von unter 50 µm, bevorzugt unter 15 µm, aufweist.

## Claims

1. A method for the basic or acidic catalyzed esterification and/or re-esterification of fatty acids and/or oils and/or fats, namely the esters of glycerol with fatty acids, by introducing low alcohols, in particular methyl alcohol, into the liquid starting product, **characterised in that** in the liquid starting product, at least one technically pure low alcohol is dispersed in the presence of a basic or acidic catalyst by means of a dispersin tool while avoiding production of a stable dispersion, **characterised in that** the phase separation is 90% completed within less than 30 minutes.

2. The method according to claim 1, **characterised in that** the phase separation is 90% completed within less than 10 minutes.

3. The method according to any one of claims 1 to 2, **characterised in that** the dispersion has a droplet size of approximately 1 µm.

4. The method according to any one of claims 1 to 2, **characterised in that** the dispersion has a droplet size of more than 3 µm, preferably more than 5 µm.

5. The method according to claim 4, **characterised in that** the dispersion has a droplet size of less than 50 µm, preferably less than 15 µm.

## Revendications

1. Procédé pour l'estérification et/ou la transestérification à catalyse basique ou acide d'acides gras et/ou d'huiles et/ou de graisses, à savoir d'esters de la glycérine avec des acides gras, par incorporation d'alcools inférieurs, en particulier le méthanol, dans le produit de départ liquide, **caractérisé en ce que** dans le produit de départ liquide, au moins un alcool inférieur techniquement pur est dispersé en présence d'un catalyseur basique ou acide à l'aide d'un appareil de dispersion en évitant de produire une dispersion stable, **caractérisé en ce que** la séparation de phase est terminée à 90% en moins de 30 minutes.

2. Procédé selon la revendication 1, **caractérisé en ce que** la séparation de phase est terminée à 90% en moins de 10 minutes.

3. Procédé selon une des revendications 1 à 2, **caractérisé en ce que** la dispersion présente une taille des gouttes d'environ 1 µm.

4. Procédé selon une des revendications 1 à 2, **caractérisé en ce que** la dispersion présente une taille des gouttes supérieure à 3 µm, de préférence supérieure à 5 µm.

5. Procédé selon la revendication 4, **caractérisé en ce que** la dispersion présente une taille des gouttes inférieure à 50 µm, de préférence inférieure à 15 µm.
